(19) Europäisches Patentamt
European Patent Office
Office européen des brevets

(11) Veröffentlichungsnummer : **0 048 927**
**B1**

(12) # EUROPÄISCHE PATENTSCHRIFT

(45) Veröffentlichungstag der Patentschrift : **30.01.85**

(51) Int. Cl.$^4$ : **C 07 C125/06**

(21) Anmeldenummer : **81107510.0**

(22) Anmeldetag : **22.09.81**

(54) **Verfahren zur Herstellung von N,O-disubstituierten Urethanen und ihre Verwendung als Ausgangsmaterial zur Herstellung von organischen Isocyanaten.**

(30) Priorität : **01.10.80 DE 3036966**

(43) Veröffentlichungstag der Anmeldung : **07.04.82 Patentblatt 82/14**

(45) Bekanntmachung des Hinweises auf die Patenterteilung : **30.01.85 Patentblatt 85/05**

(84) Benannte Vertragsstaaten : **BE CH DE FR GB IT LI NL**

(56) Entgegenhaltungen :
**DE-A- 2 004 606**
**DE-A- 2 716 540**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Buysch, Hans-Josef, Dr.**
**Brandenburger Strasse 28**
**D-4150 Krefeld (DE)**
Erfinder : **Krimm, Heinrich, Dr.**
**Heyenbaumstrasse 65**
**D-4150 Krefeld (DE)**
Erfinder : **Richter, Wolfgang, Dr.**
**Scheibler Strasse 111**
**D-4150 Krefeld (DE)**

**EP 0 048 927 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von N-substituierten Urethanen durch Umsetzung von N-mono- oder N,N'-disubstituierten Harnstoffen bzw. Polyharnstoffen mit Dialkylcarbonaten, sowie die Verwendung der erfindungsgemäßen verfahrensprodukte als Ausgangsmaterialien zur Herstellung von organischen Isocyanaten.

Die phosgenfreie Herstellung von niedermolekularen Mono- oder Bisurethanen, die sich thermisch in an sich bekannter Weise in die ihnen zugrundeliegenden Isocyanate und die ihnen zugrundeliegenden Alkohole spalten lassen, gewinnt zunehmend an praktischem Interesse, da über die Zwischenstufe der genannten Urethane ein phosgenfreier Weg zu den entsprechenden Isocyanaten eröffnet wird.

Die DE-A-2 004 606 beschreibt bereits die Herstellung von N-substituierten Urethanen durch Umsetzung von N,N'-disubstituierten Harnstoffen, beispeilsweise N,N'-Diphenylharnstoff mit Dialkylcarbonaten, beispielsweise Dimethylcarbonat unter Verwendung einer katalytischen Menge einer Base. Irgendwelche Hinweise auf die besonders gute Eignung der nachstehend näher beschriebenen erfindungswesentlichen Metallkatalysatoren sind dieser Vorveröffentlichung nicht zu entnehmen. Wie jetzt überraschend gefunden wurde, ist es möglich, die Ausbeuten der genannten Umsetzung ganz wesentlich zu erhöhen, wenn anstelle der in der Vorveröffentlichung empfohlenen basischen Verbindungen die nachstehen näher beschriebenen Metallkatalysatoren verwendet werden. Außerdem ist das nachstehend näher beschriebene erfindungsgemäße Verfahren auch unter Verwendung von N-monosubstituierten Harnstoffen oder von linearen Polyharnstoffen in ausgezeichneten Ausbeuten durchführbar.

Die erfindungsgemäße Umsetzung ist von besonderem praktischem Interesse, da

a) die Umsetzung zwischen N,N'-disubstituierten Harnstoffen und aliphatischen Kohlensäureestern gemäß folgender Gleichung (in welcher R und R' für die indifferenten Reste der Reaktionspartner stehen)

$$R{-}NH{-}CO{-}NH{-}R + R'{-}O{-}CO{-}O{-}R' \rightarrow 2\ R{-}NH{-}CO{-}OR'$$

ohne Bildung von Fremdprodukten abläuft und

b) die als Ausgangsmaterial einzusetzenden substituierten Harnstoffe bzw. aliphatischen Kohlensäureester aus wohlfeilen großtechnischen Ausgangsmaterialien zugänglich sind.

Gegenstand der vorliegenden Erfindung ist ein Verfahren zur Herstellung von N-substituierten Urethanen, dadurch gekennzeichnet, daß man N-mono- oder N,N'-disubstituierte Harnstoffe oder lineare Polyharnstoffe mit aliphatischen Kohlensäureestern in Gegenwart von anorganischen oder organischen Verbindungen des Aluminiums, Bleis, Magnesiums, Titans, Zinns, Zinks oder Zirkons als Katalysatoren zur Reaktion bringt.

Bei dem beim erfindungsgemäßen Verfahren einzusetzenden substituierten Harnstoffen handelt es sich um organische, gegebenenfalls Urethan- oder primäre Aminoendgruppen aufweisende N-mono- oder N,N'-disubstituierte Harnstoffe oder lineare Polyharnstoffe eines maximalen Molekulargewichts von 5 000, vorzugsweise 2 000, deren Harnstoff-, Urethan- bzw. Aminogruppen über Kohlenwasserstoffreste miteinander verknüpft sind, bzw. deren Harnstoffgruppen mit Kohlenwasserstoffresten substituiert sind, deren gegebenenfalls vorliegenden endständigen Urethangruppen am Sauerstoffatom mit Kohlenwasserstoffresten substituiert sind. Typische Beispiele geeigneter Harnstoffe bzw. Polyharnstoffe sind N-Methylharnstoff, N-Ethylharnstoff, N-(n-Propyl)-harnstoff, N-(iso-Propyl)-harnstoff, N-(n-Butyl)-harnstoff, N-(iso-Butyl)-harnstoff, N-Cyclohexylharnstoff, N-Benzylharnstoff, N,N'-Dimethylharnstoff, N,N'-Diethylharnstoff, N,N'-Di-(n-Butyl)-harnstoff, N,N'-Dicyclohexyl-harnstoff, N,N'-Di-Benzyl-harnstoff, N,N'-Di-(m-tolyl)-harnstoff, N-Phenylharnstoff, N,N'-Diphenylharnstoff, N,N'-Di-Carbamoyl-toluylendiamin-2,4, N,N'-Di-Carbamoyl-isophorondiamin oder Verbindungen der Formeln

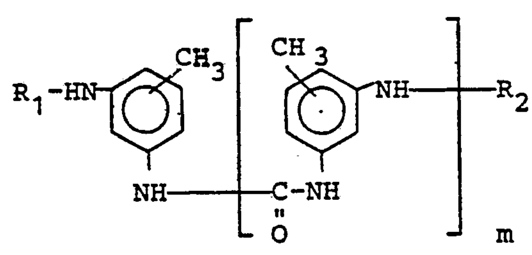

(Fortsetzung)

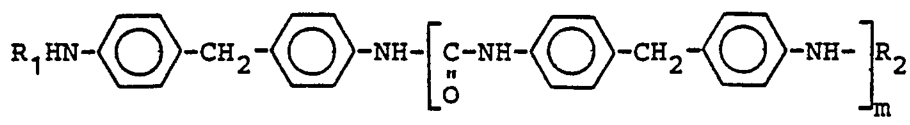

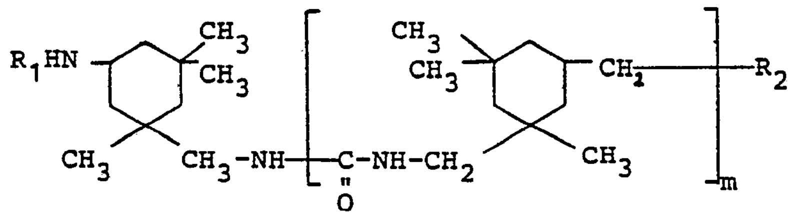

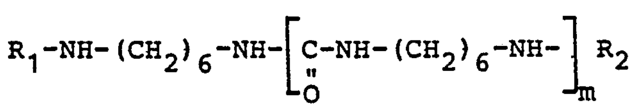

wobei m eine ganze oder gebrochene (bei statistischen Gemischen) Zahl von 1 bis 10 ist.

$R_1$ und $R_2$ für gleiche oder verschiedene Reste stehen und jeweils —H, $—COOR_3$, $—CONH_2$, $—CONHR_3$ bedeuten, wobei $R_3$ für einen einwertigen Rest, vorzugsweise einen $C_1$-$C_4$-Alkylrest, steht.

Die entsprechenden Diharnstoffe bzw. die beispielhaft genannten N,N'-disubstituierten einfachen Harnstoffe werden bevorzugt verwendet.

Geeignete Harnstoffe sind beispielsweise auch die N,N'-disubstituierten Harnstoffe bzw. die entsprechenden Polyharnstoffe, die als Nebenprodukte bei den in den US-Patentschriften 2 409 712 und 2 806 051 angegebenen Verfahren anfallen. Es können aber auch nach anderen Wegen synthetisierte entsprechend substituierte Harnstoffe (D. F. Kutepow, Russ. Chem. Rev. *31*, 633 (1962)) oder Polyharnstoffe (H. Rinke, Houben-Weyl XIV/2, 165 ff) eingesetzt werden.

Sehr gut geeignete N,N'-disubstituierte lineare Polyharnstoffe sind auch auf einfache Weise durch Umsetzung der ihnen zugrundeliegenden Diamine mit aliphatischen Kohlensäureestern der nachstehend genannten Art, wobei bei Verwendung eines Überschusses an Kohlensäureester N,N'-disubstituierte Harnstoffe bzw. Polyharnstoffe mit endständigen Urethangruppen der oben bei der Definition von $R_1$ und $R_2$ genannten Art entstehen.

Reaktionspartner für die substituierten Harnstoffe sind aliphatische Kohlensäureester wie z. B. Dimethyl-, Diethyl-, Dipropyl-, Diisopropyl, Dibutyl-, Dicyclohexyl-, Diisooctyl- und Didodecylcarbonat, gemischte Kohlensäureester wie Methyl-isopropyl- und Ethyl-phenylcarbonat, cyclische Kohlensäureester mit mindestens 6 Ringgliedern wie Trimethylencarbonat, 2,2-Dimethyltrimethylencarbonat, Bishexa-methylen-dicarbonat und lineare Polycarbonate wie Tetramethylen- und Hexamethylen-Polycarbonat. Dialkylcarbonate mit unsubstituierten $C_1$-$C_4$-Alkylresten sind bevorzugt. Die Di- und Polycarbonate werden im allgemeinen nur als Reaktionspartner für Monoharnstoffe eingesetzt. Die Dialkylcarbonate können beispielsweise gemäß DE-OS-2 748 718 aus Kohlendioxid, und Alkoholen unter Mitverwendung von Ethylenoxid als Dehydratisierungsmittel erhalten werden.

Das erfindungsgemäße Verfahren wird in Gegenwart geeigneter Katalysatoren durchgeführt. Hierzu gehören anorganische oder organische Verbindungen des Aluminiums, Bleis, Magnesiums, Titans, Zinks, Zinns oder Zirkons. Besonders bevorzugt werden organische Verbindungen der genannten Metalle eingesetzt, die in dem Reaktionsgemisch zumindest teilweise löslich sind, obwohl auch Bleidioxid zu den bevorzugten Katalysatoren gehört. Geeignete Katalysatoren sind z. B. die Oxide bzw. Hydroxide, Salze oder Alkoholate der genannten Metalle, sowie metallorganische Verbindungen der genannten Metalle, in denen der organische Rest homöopolar mit dem Metall verbunden ist, wie Zinkoxid, Zinkacetat, Zinkstearat, Zinknaphthenat, die entsprechenden Magnesiumverbindungen, Aluminiumtriethylat und -triisopropylat, Dibutylzinnoxid, Dibutylzinnchlorid, Dibutylzinndilaurat, Diacetate, Chloride oder Laurate des 2- bzw. 4-wertigen Zinns, Dioxide, Acetate oder Naphthenate des 2-wertigen Bleis, Titantetrabutylat, -tetraisobutylat, -tetraisooctylat oder -tetradodecylat oder Zirkontetraethylat, -tetrapropylat, -tetraisopropylat oder -tetraisooctylat.

Die Katalysatoren werden in Mengen von 0,001 bis 10 %, bevorzugt von 0,01 bis 3 %, bezogen auf das Reaktionsgut, eingesetzt.

Die Reaktionstemperaturen liegen im Bereich von 80-250 °C, bevorzugt bei 100-200 °C.

Das Verfahren kann bei Normaldruck oder erhöhtem Druck durchgeführt werden. Erhöhter Druck ist immer erforderlich, wenn niedrigsiedende Carbonate im mittleren oder höheren Temperaturbereich umgesetzt werden sollen.

Bei der Durchführung des erfindungsgemäßen Verfahrens wird im allgemeinen für jedes Grammäquivalent an Harnstoffgruppen mindestens 1 Mol aliphatischer Kohlensäureester eingesetzt. Im allgemeinen werden die Reaktionspartner im stöchiometrischen Verhältnis eingesetzt, da dann gemäß der Reaktionsgleichung eine glatte Reaktion ohne Nebenprodukte zum Verfahrensprodukt führt. Es kann jedoch in vielen Fällen von Vorteil sein, einen Überschuß an Kohlensäureestern anzuwenden, da sie als Lösungsmittel für schwer schmelzbare oder schwer lösliche Ausgangsstoffe z. B. aromatische Harnstoffe oder Polyharnstoffe dienen können.

Nach Beendigung der erfindungsgemäßen Umsetzung können die Reaktionsgemische durch Abfiltrieren von gegebenenfalls vorliegenden unlöslichen Nebenprodukten bzw. unumgesetzten Ausgangsmaterialien in an sich bekannter Weise destillativ oder durch Umkristallisieren aufgearbeitet werden. Die beim erfindungsgemäßen Verfahren entstehenden N,O-disubstituierten Urethane entsprechen bezügliches ihres N-Substituenten den N-Substituenten der eingesetzten Harnstoffe und bezüglich ihres O-Substituenten den Kohlenwasserstoffresten der eingesetzten Kohlensäureester. Die erfindungsgemäß zugänglichen Urethane können in an sich bekannter Weise thermisch in die ihnen zugrundeliegenden Isocyanate und die ihnen zugrundeliegenden Alkohole gespalten werden.

Die in den nachfolgenden Beispiele gemachten Prozentangaben beziehen sich auf Gewichtsprozente.

Beispiele 1-9

In einer kleinen Druckapparatur werden N,N'-Diphenylharnstoff, Diethylcarbonat und Katalysator erhitzt. Nach Erkalten wird die Apparatur geöffnet, der Inhalt zur Abtrennung von nicht umgesetztem Harnstoff filtriert und das Filtrat gaschromatographisch analysiert. Auf diese Weise werden der Umsatz an Diphenylharnstoff und die umsatzbezogene Ausbeute an N-Phenyl-O-ethylurethan bestimmt. Die folgende Tabelle gibt Auskunft über Molverhältnisse, Reaktionsbedingungen und Versuchsergebnisse.

DEC = Diethylcarbonat
DPH = Diphenylharnstoff
PU = N-Phenyl-O-ethylurethan

| Nr. | Molverhältnis DEC/DPH | Katalysator, Gew.-% bez. Reaktionsgemisch | | Temp. °C | Zeit h | Umsatz an DPH | % Ausbeute d.Th. an PU bez. Umsatz an DPH |
|---|---|---|---|---|---|---|---|
| 1 | 6/1 | $Mg(OOOC_{17}H_{35})_2$ | 0,8 | 180 | 6 | 100 | 97 |
| 2 | 6/1 | $Zn(OOOC_{17}H_{35})_2$ | 0,8 | 180 | 6 | 100 | 98 |
| 3 | 6/1 | Pb O | 0,8 | 180 | 3 | 100 | 98 |
| 4 | 6/1 | $Pb(OOOCH_3)_2$ | 0,8 | 180 | 3 | 100 | 98 |
| 5 | 6/1 | $Sn(OOOC_{11}H_{23})_2$ | 1,6 | 180 | 6 | 100 | 92 |
| 6 | 6/1 | $(C_4H_9)_2SnO$ | 0,8 | 180 | 6 | 75 | 90 |
| 7 | 9/1 | $(C_4H_9)_2SnO$ | 0,2 | 200 | 5 | 90 | 92 |
| 8 | 6/1 | $Ti(OC_4H_9)_4$ | 0,8 | 180 | 2 | 100 | 99 |
| 9 | 6/1 | Octasoligen Zirkon | 0,8 | 180 | 6 | 100 | 93 |

Beispiel 10

35,4 g (1/6 Mol) N,N'-Diphenylharnstoff, 118 g (1 Mol) Diethylcarbonat und 1,7 g Titantetrabutylat werden 7 h zum Rückfluß erhitzt, wobei die Innentemperatur 127-131° beträgt. Währenddessen geht der Diphenylharnstoff in Lösung. Durch fraktionierte Destillation des Reaktionsgemisches erhält man neben dem überschüssigen Diethylcarbonat eine bei 93-98 °C/2 mb siedende Fraktion, das N-Phenyl-O-ethylurethan. Ausbeute 53,5 g = 97,5 % d. Th.

Beispiel 11

42,4 g (0,2 Mol) N,N'-Diphenylharnstoff, 35 g (0,2 Mol) Dibutylcarbonat und 2 g Zirkontetrapropylat werden 3 1/2 h auf 190° erhitzt. Bereits nach 1 h ist der Diphenylharnstoff in Lösung gegangen. Das Reaktionsprodukt fällt nach Abkühlen kristallin aus. Man erhält nach Umkristallisieren aus Ligroin 68 g N-Phenyl-O-butylurethan entsprechend 88 % d. Th. Schmp. 60-61 °C.

Beispiel 12

37 g eines Polyharnstoffs aus 0,25 Mol 2,4-Diaminotoluol und 1,5 Mol Diethylcarbonat, 177 g (1,5 Mol)

Diethylcarbonat und 2 g Titantetrabutylat werden 23 h zum Rückfluß erhitzt (128-130° innen). Das Reaktionsgemisch wird mit Methylenchlorid verdünnt und filtriert. 24,4 g Polyharnstoff bleiben als Filterrückstand. Aus dem Filtrat erhält man 22 g m-Toluylen-bisethylurethan vom Schmp. 134-136 °C. Ausbeute 97 % bezogen auf 33 % Umsatz. Eine aus 2,4-Diaminotoluol und Chlorkohlensäureethylester hergestellte Vergleichsprobe erwies sich als identisch.

Beispiel 13

23 g Polyharnstoff aus 0,1 Mol 4,4'-Diaminodiphenylmethan und 0,6 Mol Diethylcarbonat, 34,8 g Di-n-butylcarbonat und 2 g Titantetrabutylat werden 14 h bei 192-205° gehalten. Das Reaktionsprodukt wird mit Methylenchlorid aufgekocht und der unumgesetzte Polyharnstoff (16 g) abfiltriert. Das Filtrat wird eingeengt und destilliert. Bei 92°/18 Torr geht nicht umgesetztes Dibutylcarbonat über, der Rückstand wird aus Toluol/Ligroin umkristallisiert. Man erhält 6,8 g 4,4'-Bis-(n-butoxycarbonylamino)-diphenylmethan vom Schmelzpunkt 112-113,5 °C. Ausbeute : 95 % vom Umsatz.

Beispiel 14

17,2 g (0,1 Mol) N,N'-Dibutylharnstoff, 35,4 g (0,3 Mol) Diethylcarbonat und 1 g Titantetrabutylat werden 25 h zum Rückfluß erhitzt. Man destilliert das überschüssige Diethylcarbonat ab und läßt bei 89-91°/7 Torr das N-Butyl-ethylurethan ($n_D^{20}$ = 1,429 8) übergehen. Als Rückstand bleibt Dibutylharnstoff (12,1 g). Ausbeute : 7,2 g = 99 % bezogen auf den umgesetzten Dibutylharnstoff.

Beispiel 15

42,4 g (0,2 Mol) N,N'-Diphenylharnstoff, 26 g (0,2 Mol) Neopentylglykolcarbonat und 2 g Titantetrabutylat werden 5 h auf 200° erhitzt. Man löst das Reaktionsprodukt in heißem Toluol, filtriert vom ungelösten Diphenylharnstoff (9,1 g) ab und läßt auskristallisieren. Man erhält 35 g Bisphenylurethan des Neopentylglykols vom Schmp. 136-138 °C. Ausbeute 64 % bezogen auf den Umsatz.

Beispiel 16

42,4 g (0,2 Mol) N,N'-Diphenylharnstoff, 28,8 g Hexamethylenpolycarbonat (OH-Zahl 39) und 2 g Titantetrabutylat werden 1 1/2 h auf 200° erhitzt. Das Reaktionsprodukt wird aus Toluol umkristallisiert. 3,8 g Diphenylharnstoff sind ungelöst. Man erhält 42 g Bisphenylurethan des Hexandiols vom Schmp. 165-166 °C. Ausbeute 60 % vom Umsatz.

Beispiel 17

26 g N,N'-Diphenylharnstoff, 26 g Dibutylcarbonat und 1 g Aluminiumisopropylat werden 2 1/2 h auf 190 °C erhitzt. Das Reaktionsprodukt wird in Methylenchlorid gelöst, der unumgesetzte Di-phenylharnstoff abfiltriert (3,5 g). Die Lösung wird 1 h mit 20 g säureaktivierter Bleicherde gerührt und abgesaugt. Man erhält durch Destillation bei 100-105°/0,03 Torr 27 g N-Phenylbutylurethan. Schmp. 60-61 °C. Ausbeute 57 % d. Th. = 66 % vom Umsatz.

Beispiel 18

42,4 g (0,2 Mol) N,N'-Diphenylharnstoff, 118 g (1 Mol) Diethylcarbonat und 1,7 g Bleioxid werden 7 h zum Rückfluß erhitzt. Man arbeitet wie in Beispiel 8 auf. Man erhält bei 80-85°/0,06 Torr 55 g N-Phenylethylurethan. Schmp. 47-48 °C, Ausbeute 83 % d. Th.

Beispiel 19

Ansatz 9 wird mit 1,7 g Zinkstearat als Katalysator wiederholt. Reaktionszeit 22 h. 15 g Diphenylharnstoff unumgesetzt. Ausbeute an N-Phenylethylurethan : 37 g = 56 % d. Th. = 87 % vom Umsatz.

**Ansprüche**

1. Verfahren zur Herstellung von N-substituierten Urethanen, dadurch gekennzeichnet, daß man N-mono- oder N,N'-disubstituierte Harnstoffe oder lineare Polyharnstoffe mit aliphatischen Kohlesäureestern in Gegenwart von anorganischen oder organischen Verbindungen des Aluminiums, Bleis, Magnesiums, Titans, Zinns, Zinks oder Zirkons als Katalysatoren zur Reaktion bringt.

2. Verfahren gemäß Anspruch 1, dadurch gekennzeichnet, daß man als Katalysatoren organische Verbindungen des Aluminiums, Bleis, Magnesiums, Titans, Zinks, Zinns oder Zirkons verwendet, die in dem Reaktionsgemisch zumindest teilweise löslich sind.

3. Verfahren gemäß Anspruch 1 und 2, dadurch gekennzeichnet, daß man als Katalysator Bleidioxid verwendet.

## Claims

1. Process for the production of N-substituted urethanes, characterised in that N-mono- or N,N'-disubstituted ureas or linear polyureas are reacted with aliphatic carbonic esters in the presence of inorganic or organic compounds of aluminium, lead, magnesium, titanium, tin, zinc or zirconium as catalysts.

2. Process according to Claim 1, characterised in that organic compounds of aluminium, lead, magnesium, titanium, zinc, tin or zirconium which are at least partly soluble in the reaction mixture are used as catalysts.

3. Process according to Claim 1 and 2, characterised in that lead dioxide is used as the catalyst.

## Revendications

1. Procédé de production d'uréthannes N-substitués, caractérisé en ce qu'on fait réagir des urées ou des polyurées linéaires N-monosubstituées ou N,N'-disubstituées avec des esters aliphatiques d'acide carbonique en présence de composés inorganiques ou organiques de l'aluminium, du plomb, du magnésium, du titane, de l'étain, du zinc ou du zirconium comme catalyseurs.

2. Procédé suivant la revendication 1, caractérisé en ce qu'on utilise comme catalyseurs des composés organiques d'aluminium, de plomb, de magnésium, de titane, de zinc, d'étain ou de zirconium qui sont au moins partiellement solubles dans le mélange réactionnel.

3. Procédé suivant les revendications 1 et 2, caractérisé en ce qu'on utilise le dioxyde de plomb comme catalyseur.